Europäisches Patentamt

European Patent Office

Office européen des brevets

(19)

(11) Publication number: **0 369 816**
**A2**

# EUROPEAN PATENT APPLICATION

(21) Application number: 89311949.5

(22) Date of filing: 17.11.89

(51) Int. Cl.5: **C12P 21/08, C12N 5/00,**
**G01N 33/577, A61K 49/00,**
**C07K 15/12, A61K 37/02,**
**A61K 47/00, A61K 39/395**

The title of the invention has been amended
(Guidelines for Examination in the EPO, A-III,
7.3).

(30) Priority: **17.11.88 AU 1524/88**

(43) Date of publication of application:
**23.05.90 Bulletin 90/21**

(84) Designated Contracting States:
**AT BE CH DE ES FR GB GR IT LI LU NL SE**

(71) Applicant: **THE UNIVERSITY OF MELBOURNE**
**Grattan Street**
**Parkville Victoria 3052(AU)**

(72) Inventor: **Xing, Pei-xiang**
**Flat 2, 201 Brunswick Road**
**Brunswick 3056, Victoria(AU)**
Inventor: **McKenzie, Ian Farquhar Campbell**
**359 Brunswick Road**
**West Brunswick 3055, Victoria(AU)**

(74) Representative: **Hildyard, Edward Martin et al**
**Frank B. Dehn & Co. European Patent**
**Attorneys Imperial House 15-19 Kingsway**
**London WC2B 6UZ(GB)**

(54) Monoclonal antibodies specific for human polymorphic epithelial mucin.

(57) Monoclonal antibodies which specifically bind to the amino acid sequence ALA PRO ASP THR ARG of the repeat sequence of human polymorphic epithelial mucin are disclosed. The antibodies can be used to detect mucin in biological fluids, and thus can be used to detect adenocarcinomas which are characterised by high levels of mucin production. Peptides which correspond in amino acid sequence to the epitope recognised by the monoclonal antibodies may be used as therapeutic agents in vaccines for the treatment of adenocarcinoma.

EP 0 369 816 A2

## MONOCLONAL ANTIBODIES

This invention relates to monoclonal antibodies recognizing an epitope on a mucin glycoprotein, hybridomas producing such monoclonal antibodies, uses of these monoclonal antibodies, and to vaccines containing the epitope and immunologically related peptide sequences.

Malignant and non-malignant epithelial cells secrete mucins, which are glycoproteins which exhibit considerable molecular weight polymorphism.

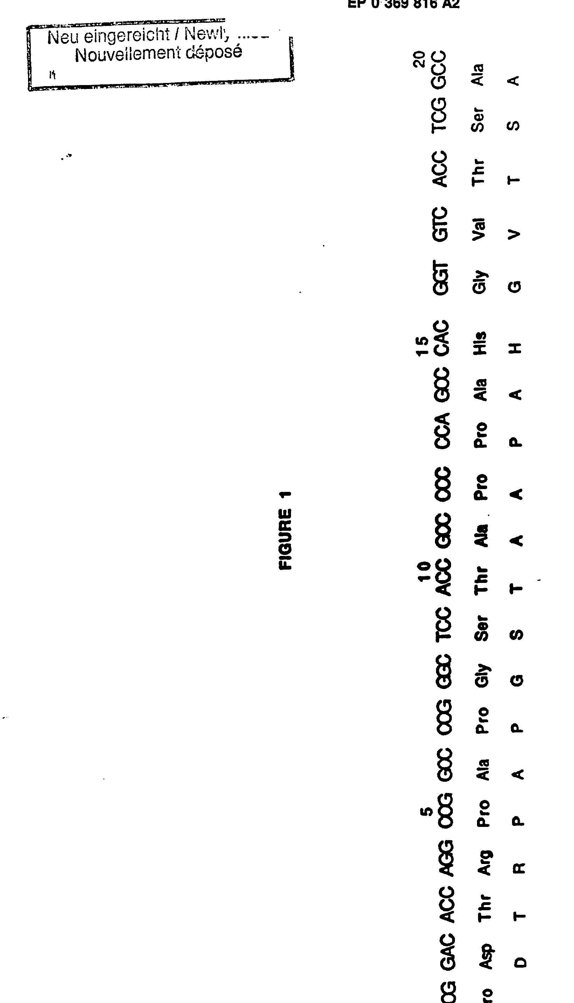

The core protein of human polymorphic epithelial mucin (HPEM -also known as PAS-O, PEM, EMA and HMFGM) has previously been shown to comprise tandem repeats of a 20 amino acid unit (Figure 1). Variation in the number of repeats between individuals in a population may account for the size polymorphism of the HPEM glycoprotein.

In individuals suffering from epithelial adenocarcinoma, such as malignant breast, ovary, colon, pancreatic or other glandular tissue cancer, large amounts of these mucins are secreted by tumour cells. These mucins may correspond to mucin normally produced by non-malignant epithelial cells, or may be aberrant mucins which may be only partially glycosylated. The amount of mucin produced in such conditions is vastly in excess of that produced by normal non-tumourigenic epithelial cells.

We have surprisingly discovered that a continuous sequence of 5 amino acids of the C-terminal end of each repeat sequence of HPEM constitutes an epitope which is detected by particular monoclonal antibodies directed against HPEM. This epitope is present on mucins expressed and secreted by epithelial adenocarcinomas, and hence using monoclonal antibodies directed against this region, epithelial adenocarcinomas may be detected.

In accordance with one aspect of the invention, there is provided a monoclonal antibody specific for the sequence ALA PRO ASP THR ARG (APDTR) of the tandem repeat sequence of HPEM. This epitope, which is present in mucin produced by both tumourigenic and non-tumourigenic epithelial tissues is bound specifically by the monoclonal antibody of the present invention. The monoclonal antibody is capable of recognising this epitope not only in the context of the mucin molecule itself, but in fragments thereof, and amino acid variants thereof. Indeed, the monoclonal antibody is generally capable of recognising this epitope regardless of flanking protein sequences. Thus, short peptides of five or more amino acids which contain this epitope may bind to the monoclonal antibody of the invention. Similarly, this epitope may be recognised by the antibody when in the context of protein molecules which differ in primary amino acid sequence (apart from that of the epitope itself) from the mucin molecule.

By varying the amino acid sequence of the epitope, we have found that the monoclonal antibody of this invention may bind to the sequence:

$A'$ P $D'$ $T'$ R

wherein:

$A'$ is A, P or S;

$D'$ is D or N; and

$T'$ is any amino acid.

The standard 1-letter amino acid code is used throughout this document. For ease of reference, this code is given in Figure 2.

On the basis of the above, the monoclonal antibody of this invention may bind specifically to the epitope ASPDR or $A'$PD$'$T$'$R (wherein these values have been given previously) in a peptide or protein, which may, for example, be represented as XASPDRX or XA$'$PD$'$T$'$RX, where X represents 1 to $1 \times 10^4$ amino acids which may be the same, or may be a combination of any known L-amino acids found in protein molecules.

The monoclonal antibody of the present invention, as discussed previously, react with mucins produced by both tumourigenic and non-tumourigenic epithelial cells, such as those from colon, ovary, lung, breast or other secretory tissue. This is to be contrasted with the monoclonal antibodies described in Australian Patent Application No. 11039/88 which react only with an aberrantly processed tumour mucin or synthetically deglycosylated mucin. These antibodies, which may be of low affinity when compared to the antibody of the present invention, would not recognise or would weakly recognise normal mucin which may be produced in large amounts by epithelial adenocarcinomas.

The monoclonal antibody of this invention is advantageous as it appears to be directed against a particularly immunogenic region of the HPEM molecule.

The origin of the monoclonal antibody of this invention, that is, species origin, is not of importance. Rather, the antibody must specifically bind to the epitope or variants thereof, as hereinbefore described. Thus, the monoclonal antibody may be of rodent, rabbit, horse, sheep, donkey or human origin or hybrids thereof.

Monoclonal antibodies may be prepared according to conventional and well known procedures, as described by Goding (Monoclonal Antibodies: Principals and Practice, 2nd Ed. Academic Press [1986]), which is incorporated herein in its entirety. Briefly, the monoclonal antibody of the invention may be produced by immunizing an animal with HPEM or HPEM that has been modified by chemical means such as deglycosylation or acetylation, or peptide segments thereof which contain the epitope APDTR. These peptides can be synthesised and optionally conjugated to a carrier protein, such as keyhole limpet hemocyanin, and used as an immunogen. The procedure is followed by obtaining immunized lymphoid cells (e.g. splenchic lymphocytes) from the immunized animals, fusing the lymphoid cells with an immortalized cell (e.g. a myeloma or heteromyeloma) to produce hybrid cells which can be propagated in culture indefinitely, and then screening the hybrid cells to identify those which produce monoclonal antibodies which react with the epitope in question. For convenience, we have routinely screened hybridoma supernatants on a peptide which corresponds to amino acids 1 to 24 of two twenty amino acid repeats of HPEM. This fragment contains the epitope APDTR.

Hybridoma production in rodents, especially the mouse, is a very well established procedure and is preferred. Stable murine hybridomas provide an unlimited source of antibody of select characteristics. Having said this, it is to be understood that this invention is not limited to murine monoclonal antibodies.

If desired, murine or other animal antibodies may be "humanized" according to the method of Riechmann et al. (Nature, Vol. 322, 24th March, 1988, pp. 323-327) which is incorporated herein in its entirety. According to these methods, recombinant DNA methodology may be used to replace the six hypervariable regions from human antibody heavy and light chain variable domains with hypervariable regions from rodent antibodies. The reshaped human antibodies would have the affinity of the original antibodies due to the presence of the original hypervariable regions but would have all other characteristics of a human antibody.

As used in this specification, the term "antibody" is intended to include fragments of antibodies bearing binding sites for the epitopes previously mentioned, such as F(ab)$^2$ fragments and single immunoglobulin variable domains, as described by Ward et al. (Nature, Vol. 341, 12th October, 1989, pp. 544-546). The teachings of this reference are also incorporated in their entirety in this specification. The antibodies of this invention may be of any isotype, such as IgM or IgG.

The monoclonal antibody of this invention may be labelled with a detectable label or reporter group such as a radioisotope, enzyme, metal, fluorophore or other directly or indirectly detectable label. Examples of enzymically detectable labels include peroxidase and urease. Examples of radioisotopes include I$^{125}$ and I$^{131}$, and isotopes of Caesium, Technecium and Indium. Examples of fluorophores include fluoroscein and rhodamine. Reporter groups may be conjugated directly to the antibodies or associated therewith, by, for example, electrostatic or other interactions. Methods for the conjugation or labelling of antibodies with reporter groups are well known in the art, and are described for example in Goding, Supra.

Monoclonal antibodies labelled with reporter groups may be used for the detection of HPEM in biological samples, such as by immunoassay, tissue staining of sections, or for administration to a patient for in-vivo localisation and visualisation of tumours using detection means such as X-ray or other imaging procedures.

In accordance with another aspect of this invention, there is provided a hybridoma which produces a monoclonal antibody which specifically binds to the amino acid sequence ASPDR of the tandem repeat sequence of human polymorphic epithelial mucin.

Hybridoma cell lines are produced according to methods well known in the art. As mentioned previously, animals are immunized with HPEM or peptide sequence thereof which contain the epitope APDTR. Immunized lymphoid cells, particularly, splenchic lymphocytes, are then fused with immortalized cells (preferable myelomas) and the resultant immortal hybridoma is maintained in culture. The monoclonal antibodies produced by the hybridomas are then tested for binding to the epitope APDTR. This may be done, for example, by synthesizing this epitope or peptide sequences containing this epitope on polyethylene pins or other supports. Binding to the immobilised peptide may then detected by ELISA, using an anti-antibody labelled with, for example, peroxidase. Those antibodies which bind the epitope in question are then selected and propagated. The hybridomas may be grown in tissue culture in the presence of an appropriate growth supporting medium, whose composition would depend upon the nature of the parent cell lines, amongst other factors, as are well known in the art. Hybridomas may also be grown within animals, such as in the peritoneal cavity. Desired monoclonal antibodies may then be harvested and purified according to methods well known in the art, such as described by Goding et al., Supra.

The hybridoma cell lines of this invention are continuous and stable cell lines which secrete desired monoclonal antibodies as previously described. The parent cell lines of the hybridoma may be derived from the same species or from different species which may be selected, for example, from mouse, rat, hampster, rabbit, human, pig, horse, sheep or goat. Particularly preferred hybridomas of the present invention are produced by hybridoma cell lines cBC1, cBC2 and cBC3, which respectively produce the antibodies BC1, BC2 and BC3.

In accordance with another aspect of the invention, there is provided a method for detecting HPEM, which comprises contacting a biological sample with a monoclonal antibody as described herein, and thereafter detecting and/or quantitating antibody binding.

Biological samples may be selected from serum, plasma, milk, urine, faeces, ascities, tissue extracts, cells and cell supernatants.

The monoclonal antibody may be labelled with a reporter group to enable detection/quantitation of antibody binding. Alternatively, agents which bind to antibodies may be employed to detect/quantitate antibody binding. Such agents include anti-antibodies which may be labelled with reporter groups or other agents such as protein A of Staphylococcus aureus.

In accordance with the method mentioned above, epithelial adenocarcinomas such as breast cancer or uterine cancer, may be detected. The indicator of a cancerous condition is elevated levels of HPEM.

Assay methods include binding antibodies to a support, adding a suspected antigen containing sample to the support, and then a second labelled or unlabelled antibody to detect the presence of HPEM. Alternatively, a method of detecting HPEM may comprises the steps of:

(i) contacting a biological sample with a monoclonal antibody which binds to the epitope APDTR;

(ii) adding the mix of step (i) to a support having immobilized thereon a peptide sequence reactive with the monoclonal antibody of step (i); and

(iii) thereafter detecting and/or quantitating antibody binding.

HPEM associated with tumour cells may be detected by injecting a patient with antibody labelled with a reporter group such as $^{125}$I or indium, and then detecting antibody binding using, for example, a scintillation camera or other imaging technique.

In another aspect of the invention, there is provided a synthetic, non-naturally occuring peptide or protein which contains or comprises the epitope APDTR or the epitope $S'A'PD'T'R$;

wherein:

$S'$ is A, D, E, F, G, K, P or S;

$A'$ is A, P or S;

$D'$ is D or N; and

$T'$ is any amino acid which occurs naturally in proteins or peptides;

or multiple copies thereof which may be represented as $[S'A'PD'T'R]^n$, $[APDTR]^n$ and $[SAPDTR]^n$; wherein n represents 1 to $1 \times 10^6$.

Such peptides or proteins may be capable of binding antibodies directed against the epitope APDTR of the HPEM molecule, and thus may be used in assays to detect this molecule.

Peptides or proteins as defined above may be used as vaccines in the treatment of adenocarcinoma. In this regard, it is believed that the epitope APDTR and possibly other sequences in the HPEM molecule may be unmasked or may become accessible to the immune system, that is, immunogenic, in a cancerous condition. Therefore, antibodies and T-cells produced on immunization with the peptide/protein defined above may, through cellular and humoral mechanisms respectively, cause destruction of tumour cells associated with these expressed epitopes.

Synthetic peptides of this invention may particularly be selected from:

p 21 - 24

p 20 - 24

p 19 - 24

p 18 - 24

p 17 - 24

p 16 - 24

p 15 - 24

p 14 - 24

p 14 - 23

p 1 - 15

p 13 - 32

TSA p 1 - 24

Ap 1 - 15

p 13 - 32

Cp 13-32

4

wherein numbers following the letter "p" refer to amino acids 1-40 of the two amino acid repeat sequence of HPEM, and wherein X represents any amino acid.

Also included within the scope of the peptides of this invention are the above peptide sequences which contain deletions, substitutions or insertions of amino acids, with the provisio that the thus altered peptides retain the ability to bind antibodies directed against the epitope APDTR.

In another aspect of the invention, there is provided a vaccine against adenocarcinoma, which comprises a peptide as previously described in assocation with a pharmaceutically acceptable carrier or excipient. Carriers and excipients include saline, albumin, dextrose and other carriers which are well known in the art and described, for example, in Remmington's Pharmaceutical Science and U.S. Pharmacopeia (1984); Mack Publishing Company, Easton, PA., which is incorporated herein by reference.

The peptides mentioned above may be conjugated or associated with an adjuvanting sequence such as PPD or MDP, or a carrier molecule, such as diptheria or tetanus toxoid.

Vaccines may particularly comprise a peptide or protein selected from: $[S'A'PD'T'R]^n$-Y

$[SAPDTR]^n$-Y

$[APDTR]^n$-Y; or

$[TSAPDTRPAPGSTAPPAHGVTSAPDTR]^n$;

wherein; n is 1 to $1\times10^6$, y is an adjuvanting sequence or a carrier molecule, and S', A', D' and T' are as previously defined.

In yet another aspect of the invention there is provided a therapeutic composition for the treatment of adenocarcinoma, which comprises a monoclonal antibody reactive with the epitope APDTR of HPEM as defined herein, either alone or in association with a pharmaceutically acceptable carrier or excipient. The monoclonal antibody is preferably of human origin or is humanized, that is, the antibody contains human constant and variable regions, and hypervariable regions from another species such as mouse or rat. The monoclonal antibody may be conjugated or associated with a cytotoxic agent such as the cytotoxic sub-unit of ricin, or tetanus toxoid, or cytotoxic drugs such as melphalan, bleomycin, andriamycin, n-acetyl melphalan, cisplatin, daunorubicin, actinomycin, 5-fluorouracil, methotrexate, cyclophosphamide and salts or derivatives thereof.

This invention will now be described by way of non-limiting example, with reference to the following Figures:

**FIGURE 1** shows the 20 amino acid repeat of HPEM;

**FIGURE 2** shows the conventional one letter amino acid code, and is taken from Lehninger, Biochemistry, 2nd Edition, Worth Publishers Inc., 1978; and

**FIGURE 3** shows the percent of "mean parent response" of replacement set analysis of SAPDTR recognized by mAbs BC1(A) and BC2(B). Each block of 20 ELISA values represents the results with peptides containing the single amino acid substitution identified by the single letter code beneath each bar. The position of the substitution in SAPDTR is indicated by an asterisk. ELISA values for the six copies of the parent sequence were averaged and taken as 100% for comparison with the values obtained with the replacement analogues.

## ABBREVIATIONS

PPD - purified protein derivative
BCG - bacillus calmette-guerin
MDP - muramyl di-peptide
HMFGM - human milk fat globule mucin
PBS - phosphate buffered saline
PVC - polyvinyl chloride.

## EXAMPLE

### 1.0 Materials and Methods:

#### 1.1 Membrane Preparations:

HMFGM (human milk fat globule membranes) were isolated from fresh human milk as previously described by Jarasch et al. (J. Cell. Biol. 73: 223-241). The crude membranes produced were then collected by centrifugation at 100,000g for 1.5 hrs. at 4°C using a Beckman L8-70 ultracentrifuge. The pellet was resuspended in 0.3M sucrose, 70mM KCl, 2mM $MgCl_2$; 10mm Tris-HCl buffer pH 7.4 and stored at -70°C. To remove the O-linked carbohydrate from the milk mucin, the molecule was treated with trifluoromethanesulfonic acid (TFMSA) for 1 hr at 20°C to produce the extensively stripped mucin. Cell surface membranes from the breast cancer cell line, ZR75, were prepared as previously described (Br. J. Surg. 75: 811-817).

### 1.2 ELISA Assays:

The anti-HMFG activity of the monoclonal antibodies was measured in a HMFGM binding assay. Crude HMFGM or p1-24 peptides were coated onto 96 well flexible PVC plates (Costar) at 25μg/ml in carbonate buffer pH 9.6 for 1 hr at 37°C. Non-specific binding sites were blocked with 1% BSA (bovine serum albumin) for 1 hr at 37°C, and plates washed with PBS pH 7.40, 05% Tween 20. Purified antibodies (0.15μg/ml - 20.00μg/ml) were reacted with the solid phase antigens for 2 hrs at 37°C and after removal of excess antibody, sheep anti-mouse immunoglobulin conjugated to horseradish peroxidase (Amersham) was added and incubated for 2 hrs at 37°C. The plates were washed again, and developed using 0.03% ABTS (2.2-axino-di-[3-ethylbenzthiazoline sulphonate])/0.02% $H_2O_2$ in 0.1M citrate buffer pH 4 and absorgance read at 405nm using an enzyne immunoassay plate reader (Titertek Multiscan MC). The reactivity of antibodies in the form of tissue culture supernatant with deglycosylated HMFGM was similarly determined using wells coated with deglycosylated HMFGM.

ELISA tests on peptides immobilized on pins were carried out as follows. Peptides immobilised on the pins were coated for 1 hr in microtitre plates containing 200μl buffer containing 1% ovalbumin, 1% bovine serum albumin, 0.1% Tween 20 in 8 PBS pH 7.2 and 0.05% sodium azide (at room temperature with agitation). The pins were then incubated overnight at 4°C in 150μl per well of antibody in the same buffer. After washing (4x10min) in PBS/Tween 20 at room temperature with agitation, the pins were incubated for 1 hr at room temperature in microtitre plates containing 150μl per well sheep anti-mouse immunoglobulin labelled with horse radish peroxidase (Amersham, UK) in PBS/Tween 20 (1:400). The pins were then washed with vigorous agitation (4x10min) in PBS/Tween 20 at room temperature and incubated at room temperature in the dark in microtitre plates containing 150μl per well of substrate 0.05% ABTS (2,2-azino-di-(3-ethylbenzithiazolinesulfone sulfonate) (Amersham UK) in 0.1M phosphate 0.08M citrate buffer pH 4.0 containing 0.04ml $H_2O_2$ per 100ml buffer. Incubation was stopped when the plates appeared to have sufficient colour by removing the pins, after which 50μl from each well was transferred to another microtitré plate and the absorbance read immediately at 405nm using a Titertek Multiscan MC plate reader.

The binding of the purified antibodies (2.3μg/ml -300.0μg/ml) to mucins derived from a breast cancer cell line was assessed by the Z75 membrane binding assay ZR75 membrane was coated and dried onto PVC plates and the direct ELISA performed as for HMFGM binding assay. MSA activity in serum samples was determined by a competitive enzyme immunoassay where serum was used to inhibit the binding of monoclonal antibody 3E1.2 to a ZR75 membrane preparation which contains MSA. Their ability to inhibit binding was expressed as inhibition units (I.U.) on a scale from 1-10,000 I.U. Patients with breast cancer tend to have levels of 300-10,000 I.U. , and normal individuals low levels (<300 I.U.).

### 1.3 Immunoperoxidase Staining:

Immunoperoxidase staining on sections of formalin fixed tissues was performed according to the procedure of Stacker (J. Natl. Cancer Inst. 75: 801-811 [1985]). The staining was graded according to the percentage of cells stained: 0% (0), <25% (1), 25-50% (2), 50-75% (3), >75% (4) and the intensity of staining.

### 1.4 Collection of Serum Samples and Milk:

Serum samples from patients with disseminated breast cancer were obtained from the Royal Melbourne Hospital, Melbourne. The criteria for staging is in accord with accepted defintions (Beahrs et al., American Joint Committee on Staging - Manual for Staging of Cancer, 2nd Ed., Philadelphia: Lippincott [1983]).

Normal serum samples were obtained from apparently healthy blood donors at the Red Cross Blood Bank (Melbourne, Australia). Serum samples and milk were stored frozen at -70°C.

## 1.5 Serum Assays using Double-Determinant Radioimmunoassay:

Microtitre wells were coated with various monoclonal antibodies (100μl/well) in a 0.5M carbonate buffer pH 9.6 and wells were blocked with a solution of 2% bovine serum albumin and 2% normal mouse serum. Serum samples were diluted 1:4 in buffer (PBS/0.05% Tween 20) and added to wells for 4 hrs at 4°C; the plates were then washed and $^{125}$I-labelled antibodies were added at $2.5 \times 10^5 - 3.0 \times 10^5$ cpm/50μl/well (equivalent to 140-180 ng/well) for 4 hrs at 4°C, after which the plates were washed and the radioactivity in each well determined. Radioactive counts were converted to units by comparison with a calibration curve obtained with a reference pool of serum samples from patients with metastatic breast cancer who had high MSA (an antigen associated with breast cancer, which is associated with the HPEM molecule) levels (>10,000 I.U.). Levels of antigen in the serum were assigned by comparison of radioactive count with a panel of sera from normal individuals which was arbitrarily assigned antigen levels (means ± 2 SD) of 100 units for BC3/BC2 test and 80 units (means ± 2SD) for BC3/3E1.2 test respectively.

## 1.6 Peptide Synthesis:

Peptides were produced using an Applied Biosystem Model 430A automated peptide synthesizer (Foster City, CA., U.S.A.), based on the standard Merrifield solid phase method (Hodges, R.S. and Merrifield, R.B. [1975] Biochem. 65: 241). Chain assembly occured in a stepwise procedure by sequential deprotection with trifluoroacetic acid, neutralization with disopropylethylamine and coupling with dicyclohexylcarbodimide via the performed amino acid symmetric anhydride. Boc and Aoc amino acid derivaties and polystyrene resin were purchased from Protein Research Foundation, Japan. The process was monitored with ninhydrin reaction (Sasrin et al. 1981, Anal. Biochem 117: 147) and peptides were cleaved with anhydrous trifluoromethanesulfonic acid. Crude peptides (p1-24, p1-15, p16-24) were purified by reversed phase high pressure liquid phase chromatography (HPLC) (Waters Association, Milford, MA., U.S.A.) using 0.1% trifluoroacetic acid-water-acetonitrite gradients and a C8-aquapore Rp-300 column (Brownlee, U.S.A.). Synthetic peptides p1-24, p1-15 and p16-24 were approximately 90% pure as judged by analytical reverse phase HPLC and by amino acid sequence analysis. Peptides are named either by position number in the peptide p1-40 consisting of two twenty amino acid repeats or by individual amino acid name combined with the following peptide name. Some shorter peptides i.e. p21-24, p20-24, p19-24, p18-24, p17-24, p15-24 p14-24, p14-23, pA+p1-15 were not purified. An irrelevant peptide, T4N1 (corresponding to the amino-terminus of mouse CD4) was used as the control. The peptides are named according to their position in the 1-24 amino acid peptide (Table 2).

## 1.7 Peptide Synthesis Using Polyethylene Pins:

Peptides were synthesised using a commercially available kit (cambridge Research Biochemicals, Cambridge, UK). The synthesis was performed on solid polyethylene pins in an 8x12 arrangement using pentafluorphenyl (pfp) active esters of fluorenylmethyloxycarbonyl (Fmoc)-L-amino acids. Exceptions to this were serine and threonine where the pfp was replaced by (ODhbt). Side chain protecting groups used were: trityl for cysteine, t-butyloxy for aspatic and glutamic acids, t-butyloxycarbonyl for histidine and lysine, 4-methoxy-2, 3 5-trimethyl for arginine and t-butyl for serine threonine and tyrosime. The pins were supplied with as Fmoc β-alanine residue pre-coupled via hexamethylene diamine and acrylic acid to irradiated polyethylene. As the first stagae of synthesis this Fmoc protecting group was removed (deprotection) by mild base cleavage with 20% (v/v) piperidine (Applied biosystems, Foster City, CA) in dimethylformamide (DMF) (Peptide Synthesis Grace, Auspep, Australia). All other chemicals used were of Analar quality or greater and were used without further purification. Following deprotection and washing, the appropriate Fmoc-amino acid ester was activated by dissolving to a concentration of 30mM in DMF containing 30mM 1-hydroxybenzotriazole. Aliquots (100μl) of the solution were immediately dispensed into the polypropylene trays provided. Amino acid coupling was for 18 hrs at room temperature. Deprotection and coupling steps were repeated until hexameric peptides were synthesised. The N-terminus of each peptide was then acetylated by reaction with DMF:acetic anhydride:disopropylethylamine (5:2:1 v/v/v) and finally side-chain

EP 0 369 816 A2

deprotection and neutralisation achieved by reaction with trifluoroacetic acid:phenol:ethanedithiol (95:2.5:2.5 v/w/v) followed by washing in dichloromethane:diisopropylethylamine (95:5 v/v). The pins were washed in methanol for 18 hrs and dried in vacuum.

Successful synthesis on each 8x12 pin block was determined immunochemically by the synthesis of positive and negative control pins and comparison of their reactivity with test mAb to pins on which the control peptides had been synthesised by the manufacturer. In each case the synthesised control pins showed the same reactivity as those provided by the manufacturer.

Prior to initial use and before every re-use, the pins were subjected to a disruption procedure to remove bound antibody. The pins were sonicated in a buffer of 1 sodium dodecyl sulphate, 0.1% 2-mercaptoethanol, 0.1M sodium dihydrogen orthophosphate prewarmed to 60°C for 30 min then washed with hot distilled water and finally with boiling methanol.

## 1.8 Preparation of BSA - peptide conjugate:

Peptides were conjugated to bovine serum albumin (BSA) using a modification of the glutaraldehyde coupling method (Brian et al., 1985, J. Immunol. Methods 78: 59-69). One ml of peptides (1mg/ml) and 0.1ml 2% BSA were mixed with 0.5ml of 0.25% glutaraldehyde at ambient temperature for 6 hrs and the mixture dialysed against PBS overnight at 4°C.

## 1.9 Binding of Antibodies to Synthetic Peptides:

Synthetic peptides or BSA-peptide conjugates were coated onto 96 well flexible PVC plates (Costar) at 20μg/ml on 0.05M carbonate buffer pH9.6 for 2 hr at 37°C. After washing with PBS-0.05% Tween 20 purified monoclonal antibodies were added to each well at a double dilutions (150μg/ml - 75ng/ml) and incubated at ambient temperature. After 2 hrs plates were thoroughly washed and sheep anti-mouse immunoglobulin labelled with horse radish peroxidase was added to plates, and incubated for 1 hr at ambient temperature. The plates were washed and the bound antibody was detected by the addition of 0.03% ABTS (2.2-azino-di-[3-ethyl-benzthiazolinesulfone sulphonate]) in 0.1M citrate buffer pH4, containing 0.02% $H_2O_2$. The absorbance was measured at 405nm using a Titertek Multiskan MC. As a positive control HMFGM was coated onto the plates at 10μg/ml.

Peptides were examined in liquid phase (competition or inhibition assay) by incubating antibody with dilutions of peptide (o.4x10$^{-3}$ - 0.8 mM) or with HMFG (0.4μg/ml - 800μg/ml) for 1 hour at room temperature prior to adding to plates which were coated with 10μg/ml of HMFG. Inhibition of antibody binding to HMFG was quantitated with sheep anti-mouse immunoglobulin labelled with horse radish peroxidase.

## 1.10 Affinity Measurements (Ka):

The affinities of monoclonal antibodies BC1 and BC2 for their binding to HMFG or peptide p1-24 were determined by a radioimmunoassay method. Assays were carried out with serial dilutions of $^{125}$I radiolabelled antibodies BC1 and BC2 in the PVC 96 well plates coated using HMFG or p1-24. The counts per minute of antibody binding were measured in a gamma counter (LKB Wallac 1260, Finland). To calculate Ka the method of Scatchard was used.

## 1.11 Antibodies:

Analysis of antibody class was determined by Ouchterlony gel immunodiffusion with class-specific rabbit antisera (Serolec, England). Monoclonal antibodies were purified from ascites fluid: IgG antibodies by Protein A-Sepharose (Pharmacia Inc., Piscataway, N.J.) and IgM antibodies by recipitation with 33% saturated ammonium sulphate, and dialysis against phospate buffered saline (PBS) pH 7.4 at 4°C. Monoclonal antibodies referred to herein included 3E1.2 developed against primary breast carcinoma (Stacker et al., 1985, J. Natl. Cancer Inst. 75: 801-811), 5C1 (IgM) which is reactive with colon carcinoma, and RCC-1, an IgG2a which is reactive with an antigen for Mr95,000 (Thompson et al. 1983, JNCI. 70: 409-419).

8

## 2.0 Monoclonal Antibody Production and Analysis:

(CBA x BALB/c)F$_1$ mice (female, age 8 weeks) were immunized by intraperitoneal injection of 200$\mu$g HMFGM emulsified in complete Freunds adjuvant, three times at 3-6 week intervals, and boosted intravenously with 200$\mu$g HMFGM and 100$\mu$l human milk 3 days before fusion. Spleens were removed and teased in 0.83% NH$_4$Cl to remove red cells, the suspension was decanted and centrifuged at 200Xg for 5 minutes, and the pellet was washed twice in serum-free DMEM. The spleen cells (4x10$^7$) were mixed with 2x10$^7$ washed NS-1 myeloma cells and centrifuged at 200Xg for 5 minutes, and the supernatant was discarded. The pellet was then resuspended in 0.8 ml of 50% (wt/vol) polyethylene glycol(Mr 1,500; BDH Chemicals, Poole, England), iubated at 37$^\circ$C for 1 minute, and centrifuged at 200Xg for 5 minutes; the supernatant was then removed and the pellet resuspended in 10 ml of DMEM. The mixture was recentrifuged, and the pellet was slowly resuspended to 30 ml in DMEM with 10% FCS containing B10.A strain thymus cells as feeder cells. The suspension was distributed into 288 wells (three microtitre plates; Cooke Laboratories, Singapore). Fresh DMEM supplemented with HAT (Sigma Chemical Co., St. Louis, Mo.) was added on days 1, 2, 4, 7 and 9. On day 10 all wells were individually screened for antibody production.

Tissue culture supernatants were screened for antibodies against HMFGM with an ELISA test. The positive hybridomas were then cloned and recloned by limiting dilution and then grown as ascitic tumours in pristane-primed (CBA x BALB/c) F1 mice. Monoclonal antibodies were purified from ascites fluid as described in Section 1.11. From the hybrid clones produced, three antibodies, BC1 (IgG3), BC2 (IgG1) and BC3 (IgM), were selected on the basis of strong reaction with the immunogen (HMFGM) as determined by ELISA and which did not show any reactivity with lymphocytes or granulocytes by Fluorescence Activated Cell Sorter San (Becton - Dickinson, Mountain View, CA, U.S.A.) (Teh et al., 1988, J. Immunol. Methods 110: 101-109). These antibodies were then screened against formalin-fixed, paraffin -embedded sections of breasat carcinoma, normal breast and benign breast tumours. The monoclonal antibodies BC1, BC2 and BC3 were respectively produced by hybridomas cBC1, cBC2 and cBC3. These hybridomas and monoclonal antibodies were maintained in the culture collection of the Research Centre for Cancer and Transplantation at the Department of Pathology, The University of Melbourne, Parkville 3052, Victoria, Australia.

## 2.1 Tissue Distribution:

Table 1 shows the reactivity of the 3 antibodies with various tumours. The antigens detected by antibodies BC1, BC2 and BC3 survived formalin fixation and paraffin embedding. Of the 44 breast carcinomas examined, 42/44, 40/44 and 43/44 were positive for antibodies BC1, BC2 and BC3 respectively. Breast carcinomas (infiltrating and in situ) showed staining of both membranes and cytoplasm as well as luminal membrane staining and peripheral accentuation in some cases (not shown). In the majority of cases (~70%), the percentage of carcinoma cells stained was >75% and the staining intensity was strong. By contrast, BC1, BC2 and BC3 reacted weakly with the apical membrane of normal breast epithelial cells in acini and ducts. There was a heterogeneous reactivity with other normal tissues but, in general, any reactivity with normal tissues was weak. In particular, none of the antibodies shows any reactivity with connective tissue, muscle, or lymph nodes, rendering them potentially useful for in vivo immunolymphoscintigraphy. However, some very minor reactivity was detected in some normal epithelial tissues especially pancreas, kidney and lung.

The cell distribution of antigens reactive with BC1, BC2 and BC3 were studied in 3 primary infiltrating duct carcinomas of the breast and the corresponding metastatic lesions. There was variation in the percentage of carcinoma cells stained and the intensity of staining between the primary lesion and the corresponding metastatic lesion, in accord with findings of antigenic heterogeneity between tumour masses. The proportion of carconima cells stained and the intensity of staining in a tumour also varied between antibodies.

## 2.2 Identification of the Antigens Recognized by the Monoclonal Antibodies:

To confirm that the antibodies recognized mucins, they were tested in ELISA experiments with preparations of HMFGM and ZR75 membrane (a cell membrane preparation which does not contain HPEM), and in immunoblotting experiments with serum of patients with disseminated breast cancer. It was found that although the antibodies differed from each other in their ability to bind mucin preparations from different sources, they all recognized high molecular weight (230,000-330,000) molecules; those present in the serum at least partially co-migrated with those recognized by antibody 3E1.2, a monoclonal antibody directed against HPEM. In addition, a smaller molecular weight species (Mr ~ 110,000) was also detected in HMFGM by antibodies BC1,BC2 and BC3. Because the epitopes recognized by antibodies BC1, BC2 and C3 differed from that recognized by antibody 3E1.2 in that 3E1.2 does not react with the intact mucin (HMFGM) processed by the lactating breast and yet reacts with similar high molecular weight molecules in the serum, it was appropriate to examine their reactivity with milk mucin core protein.

## 2.3 Reactivity of Monoclonal Antibodies to the Milk Mucin Core Protein:

To remove the O-linked oligosaccharides, HMFGM was treated with TFMSA (Edge et al., 1981, Anal. Biochem. 118: 131-137) which has been shown to be effective in removing sugars from mucin without damaging the protein core. After treatment for 1 hr at room temperature; the high molecular weight bands had disappeared resulting in polypeptide bands of MW ~ 70,000. BC1, BC2 and BC3 reacted strongly with the mucin stripped of its carbohydrate, as well as intact mucin (HMFGM) despite the fact that they were developed using intact mucin (from the milk fat globule) as immunogen. In contast, antibody 3E1.2, did not react or reacted only weakly with either glycosylated or deglycosylated HMFGM. These findings suggest that BC1, BC2 and BC3 bind to epitopes on the core protein.

## 2.4 Reaction of Antibodies with Synthetic Peptides in the Direct Binding Assay:

The 20 amino acid sequence of a first repeat of HPEM plus 4 amino acids of the next repeat is shown as p1-24 in Table 2. Peptides of varying length were synthesized (Table 2) and an irrelevant peptide T4N1 (as a negative control) was also included as a control. The binding of the antibodies to these peptides was tested in the solid phase direct binding assay. Positive and negative reactions of antibodies BC1, BC2 and BC3 with synthetic peptides were demonstrated.

In the direct binding assay the three antibodies (BC1, BC2 and BC3) bound to p1-24 and to p14-24 (the "right hand" 11 - mer peptide [14]AHGVTSAPDTR[24]) but not to the "left hand" 15 mer ([1]PDTRPAPGSTAPPAH[15]). There was also a reaction with the p15-24 (right hand 10 mer). It was noted that PDTR was shared by both of these peptides and as one was reactive and the other not, presumably the 4-mer itself was not a reactive epitope. However, there was a possibility that the amino acid adjacent to PDTR in the repeating peptide (alanine) could account for the reactivity and so p1-15 was resynthesised with A as the N-terminal amino acid (A + p1-15). The addition of the single amino acid was sufficient for p1-15, previously non-reactive, to become reactive, indicating that APDTR could be the antibody reactive epitope. On this basis, six short peptides were synthesised, each differing by one amino acid added to the N-terminal end (PDTR → p16- 24, a 9 mer). None of these reacted in the direct binding asay. Thus APDTR appeared to be a reactive epitope provided it was adjacent to other amino acids although the nature of these and on which flank did not appear to be important. This suggested that the conformation of the peptide, at least in the solid phase, required an adjacent peptide and on this basis the peptides were attached to a carrier protein (BSA). Their reactions were determined in a direct binding assay and all the peptides were also tested in a liquid phase competition assay.

Although not described in Table 2, it was noted that several other anti-HMFG antibodies (CC-2, CC-3, CC-4, 2B4 and 41.3) and monoclonal antibodies against colon cancer (XPX-1, Lk-4, SMA-1, 17C4, I-1 and GJT-13) were non-reactive with the peptides. Presumably they react with carbohydrate dependent epitopes or with epitopes whose tertiary structure is not represented in the linear peptides.

No antibody reacted with a "control", CD4 peptide (T4N1).

## 2.5 Further Reactions of Antibodies with Synthetic Peptides in Direct Binding and Inhibition Binding Assays:

Various synthetic peptides based on the amino acid sequence of HPEM and variants thereof were synthesized and tested for binding with antibodies BC1, BC2 and BC3 in direct binding and inhibition assays. Results are shown in Table 3.

As is evident from Table 3, APDTR inhibited binding of BC1, BC2 and BC3 to HMFGM in the liquid

EP 0 369 816 A2

phase competition (inhibition) ELISA assay, but more weakly than p1-24. No five amino acid analogue inhibited binding to HMFGM. In the peptide of p13-32, where the epitope was in the centre of the peptide (this corresponding to a region of hydrophobicity) increased antibody binding compared to p1-24 was observed.

Analogues Ap1-15, Ap1-24, TSAp1-24 and C-p13-32 all displayed strong binding to the antibodies BC1, BC2 and BC3 which was greater than binding to p1-24. The result did not simply increase two times in the reaction, as one might expect from peptides containing two epitopes in one peptide. It was much more than two times stronger TSAp1-24 reacted with BC1 BC2 and BC3 more strongly than A-1-24. This result suggests that adding TSA to p1-24 makes the whole peptide conformation change in a way which is more suitable to reaction. This makes it possible to significantly enhance antigenicity by synthesizing a peptide with repeating amino acid sequences corresponding to the epitope APDTR or variants thereof as described herein. These findsings are of considerable importance, as highly immunogenic peptides/proteins may be produced which can then be used in the diagnosis and/or treatment of adenocarcinoma. The presence of cysteine at the N-terminal end of the peptide C-p13-32 may increase reactivity of the peptide through the formation of dimers, trimers, etc., or other cyclization. There was no binding to control peptide T4N1.

## 2.6 Reaction of BC1, BC2 and BC3 with BSA - Peptide Conjugates:

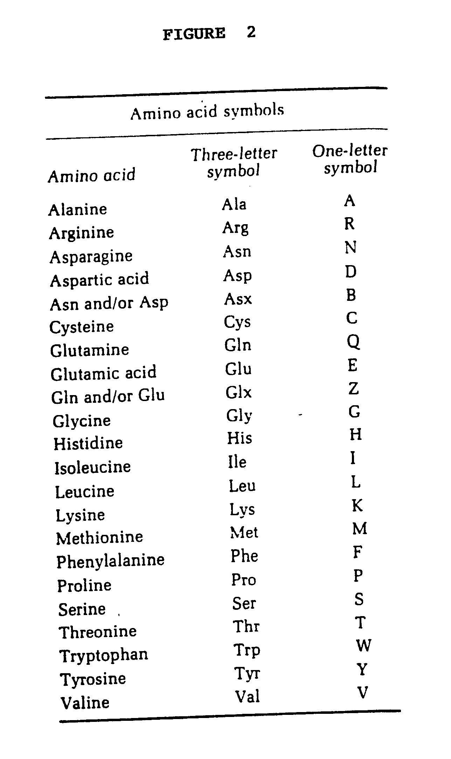

The BSA-peptide conjugates of all the peptides were prepared and tested in the direct binding assay (Figure 2). The results confirmed APDTR to be the reactive epitope but not PDTR or amino acids in the left hand -1-15 (although a weak reaction has noted with BC1, not shown). Thus the results showed BSA-p21-24, BSA-p1-15 and BSA-T4N1 were still non-reactive, but BSA-p20-24, BSA-p19-24, BSA-p18-24, BSA-p17-24 and BSA-p16-24 were positive whereas they were non-reactive with the non-conjugated peptides. In addition, BSA-peptide conjugates, BSA-p15-24, BSA-p14-24; and BSA-p1-24 were positive in both assays. It was clear that all 3 antibodies reacted with the 5 mer APDTR.

## 2.7 Competition Assay:

As the conformation of the peptides could be different in liquid that in solid phase the reaction of peptides in a solution ( rather than in solid phase as described above in the direct binding assays) competitive inhibition assay were examined. In these studies the soluble peptides were tested for their abaility to inhibit the binding of HMFG. Results are shown in Figure 2 as "Inhibition Test". The inhibition test results are largely comparable to those obtained with direct binding to peptide conjugates. However, it was noted that no inhibition of BC3 occured with the 5 mer (p20-24 APDTR) or 6 mer (p19-24 SAPDTR). It is likely that BC3 being an IgM is less effectively inhibited by a small peptide in solution than the smaller IgG antibodies.

## 2.8 Antibody Binding to Synthetic Peptides on Pins:

Peptides were synthesized on polyethylene pins as described in Section 1.7.

The peptide APDTR bound to the pin was specifically bound by antibodies BC1, BC2 and BC3. The peptides PDTR and TSAPDTR were also synthesized on polyethylene pins. The antibodies did not react with PDTR, but reacted very strongly with TSAPDTR. These results confirm that APDTR is the epitope recognized by BC1, BC2 and BC3.

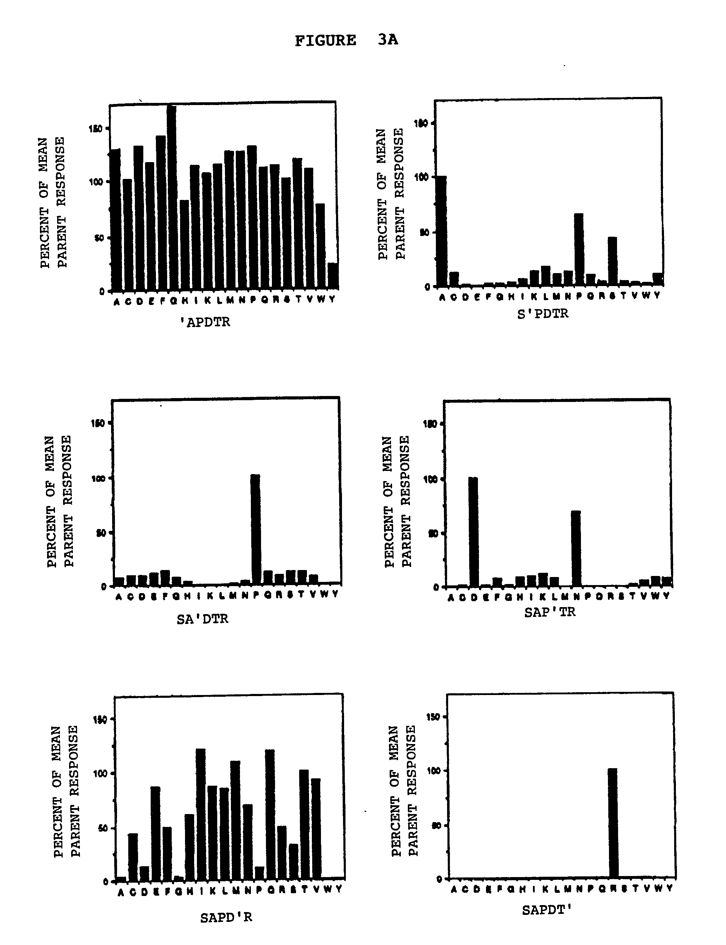

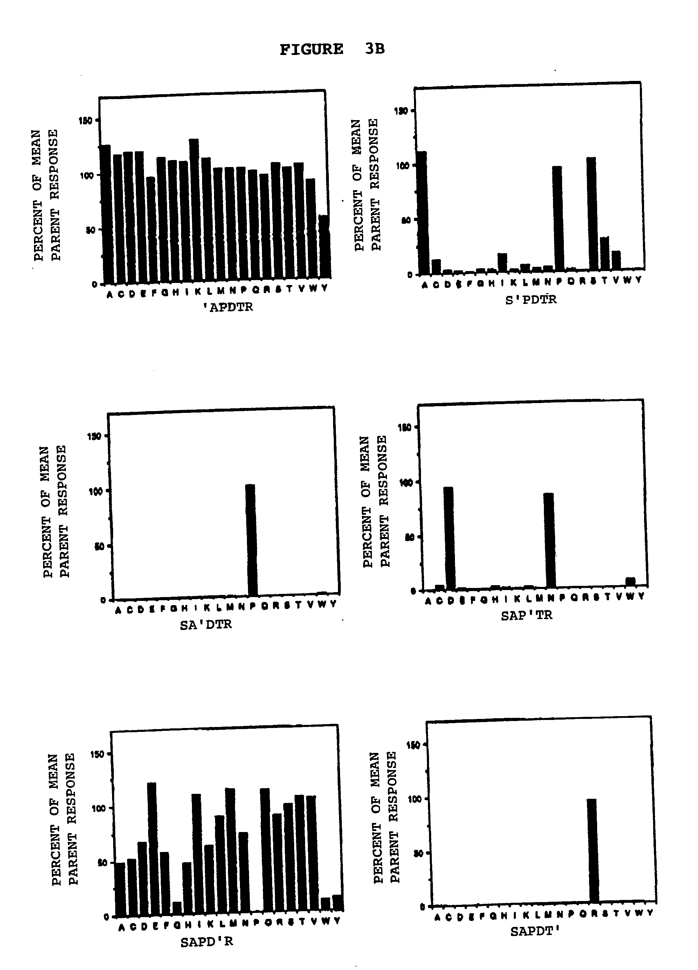

To determine whether any of the amino acids in the epitope APDTR can be varied without destroying antibody binding the 6 mer SAPDTR was synthesized with all 20 variations of each amino acid in each position, i.e. 120 peptides were synthesized on polyethylene pins and antibody binding detected. Results are shown in Figures 3A and 3B.

Figure 3A shows the results of the replacement set in an ELISA assay using BC1. Clearly the residues P and R are essential to antibody binding as replacement by any other amino acid results in an essentially complete loss of binding to the peptide. The residue D is similarly almost completely irreplaceable except by the related amino acid N. The S residue is completely replaceable by virtually all amino acids, providing futher confirmation that APDTR is the minimum epitope. A shows limited replaceability by P + S while T shows quite a high degree of replaceability. As T may be a glycosylation site, it is possible that the amino acid, as such, does not contribute to the epitope.

11

Figure 3B shows the results in the same ELISA assay for BC2. There results provided to be very similar to those of BC1. Completely irreplaceable residues were P and R. A and D showed limited replaceability. Relatively universal replaceability was shown for for S and T with some notable exception in the latter case.

The results shown in Figure 3 indicate the epitope $S'A'PD'T'R$; wherein $S'$ is A, D, E, F, G, K, P or S; $A'$ is A, P or S; $D'$ is D or N; and $T'$ is any amino acid which occurs naturally in proteins or peptides; binds antibodies more strongly than does the naturally occuring epitope APDTR. This result is of considerable significance, as peptides/proteins containing or consisting of these sequences or repeats thereof, can be used in assays for the detection of HPEM, or as vaccines in combination with suitable carriers or adjuvants, as are well known in the art.

### 3.0 Reproducibility of Antibody Production:

Monoclonal antibodies reactive against the epitope ASPDR of human HPEM may be repeatably produced. This was demonstrated by carrying out repeated fusions according to the procedures of Section 2.0. Hybridomas were then tested for antibody production by screening against the peptide APDTR bound to polyethylene pins according to the procedures of Section 1.2. Hybridomas secreting antibodies reactive with the epitope APDTR were repeatedly detected. As an example, in one fusion, of the 136 hybridomas screened, 5 produced antibody reactive with the epitope APDTR.

### 3.1 Detection of Circulating Mucin using Double-Determinant Immunoassay in Breast Cancer Patients and Normal Subjects:

A double-determinant immunoassay was employed to confirm the co-expression of monoclonal antibody (BC1, BC2, BC3 and 3E1.2) defined epitopes on the same molecules in the serum. It was of interest to determine if the BC antibodies would complement 3E1.2 (MSA assay -the MSA antigen is associated with breast cancer and is detectable in the serum of patients with such a condition. The antigen is HPEM and is detected by the antibody 3E1.2) in serum test. As shown in Table 4, $^{125}$I-labelled BC1, BC2, BC3 and 3E1.2 antibodies bound to wells coated with BC3 antibody and to which the sera of patients with disseminated breast cancer had been added, indicating that some or all of the epitopes defined by BC1, BC2 and 3E1.2 antibodies are co-expressed with BC3 -defined epitopes upon at least a proportion of the BC3 antigenic molecules in the sera.

From preliminary tests on sera from patients with breast carcinoma and normal subjects, it was apparent that although all antibodies detected elevated serum antgen levels in some patients with metastatic breast cancer, the accuracy varied between the antibodies. The BC3/BC2 and BC3/3E1.2 (BC3 antibody for antigen capture in both cases) gave the best results (Table 5), detecting elevated antigen levels in 87% and 81% respectively of sera from patients with metastatic breast cancer. The sensitivity of BC3/BC2 test (87%) or BC3/3E1.2 test (81%) was lower than that of the MSA test but the specificities in normal subjects were comparable (Table 5).

Of the two tumour patient sera (metastatic) which tested negative with the MSA test, both were positive with BC3/3E1.2 or BC3/BC2 tests. Thus, by combining MSA test with BC3/BC2 or BC3/3E1.2 test, 100% of patients with disseminated breast cancer tested positive, with no deterioration in the specificity (Table 3). Thus, while both the BC3/BC2 and BC3/3E1.2 assays detected fewer positives than the MSA test in patients with disseminated breast cancer, both tests appeared to be useful in complementing MSA test.

### 3.2 The Affinity of BC1 and BC2:

The affinity of the BC1 and BC2 antibodies for HMFG and the peptide p1-24 was examined. BC2 (Ka $1.7 \times 10^{10}$) had about 10 fold higher affinity for HMFG than did BC1 (Ka $1.7 \times 10^9$). A similar finding occured on peptide p1-24 (about 3 fold, BC1 Ka $8.0 \times 10^8$, BC2 Ka $2.5 \times 10^9$). The affinity of BC1 and BC2 on the p1-24 peptide was lower than HMFG. BC3 affinity was not measured as it is an IgM.

## Table 1
Immunoperoxidase staining on section of formalin - fixed, paraffin - embedded tissues.

| | | No. of positive staining/total tested[a] | | |
|---|---|---|---|---|
| | | BC1 | BC2 | BC3 |
| **Breast Tumours** | | | | |
| Infiltrating ductal carcinoma - | Well-differentiated | 2/2 | 2/2 | 2/2 |
| | Moderately-differentiated | 9/9 | 9/9 | 9/9 |
| | Poorly-differentiated | 27/29 | 25/29 | 28/29 |
| Infiltrating lobular carcinoma | | 3/3 | 3/3 | 3/3 |
| Carcinoma in situ | | 1/1 | 1/1 | 1/1 |
| Carcinoma metastases | | 3/3 | 3/3 | 3/3 |
| Benign lesions[b] | | 2/3 | 2/3 | 3/3 |
| **Lung Tumours** | | 5/7 | 3/7 | 5/7 |
| **Uro-genital tract tumours** | | | | |
| Ovarian carcinomas | | 3/3 | 3/3 | 3/3 |
| Renal cell carcinomas | | 4/4 | 3/4 | 3/4 |
| **Gastrointestinal tract tumours** | | | | |
| Gastric carcinomas | | 2/2 | 2/2 | 2/2 |
| Colorectal carcinomas | | 5/7 | 5/7 | 6/7 |
| Pancreatic carcinomas | | 3/3 | 3/3 | 1/3 |
| **Lymphoma** | | 0/1 | 0/1 | 0/1 |

EP 0 369 816 A2

| | 55 | 50 | 45 | 40 | 35 | 30 | 25 | 20 | 15 | 10 | 5 |
|---|---|---|---|---|---|---|---|---|---|---|---|
| Neuroblastoma | | | | | | | | 0/3 | 0/3 | | 0/3 |
| Normal Tissues | | | | | | | | | | | |
| Breast | | | | | | | | 2/4 | 1/4 | | 3/4 |
| Lung | | | | | | | | 2/2 | 2/2 | | 2/2 |
| Ovary | | | | | | | | 0/1 | 0/1 | | 0/1 |
| Cervix | | | | | | | | 0/2 | 0/2 | | 0/2 |
| Colon | | | | | | | | 0/7 | 0/7 | | 0/7 |
| Small bowel | | | | | | | | 1/1 | 0/1 | | 1/1 |
| Pancreas | | | | | | | | 3/3 | 3/3 | | 3/3 |
| Spleen | | | | | | | | 0/1 | 0/1 | | 0/1 |
| Liver | | | | | | | | 0/2 | 0/2 | | 0/2 |
| Gall Bladder | | | | | | | | 0/2 | 0/2 | | 0/2 |
| Skin | | | | | | | | 1/1 | 1/1 | | 1/1 |
| Lymph node | | | | | | | | 0/3 | 0/3 | | 0/3 |
| Thyroid gland | | | | | | | | 0/2 | 0/2 | | 0/2 |
| Adrenal gland | | | | | | | | 0/1 | 0/1 | | 0/1 |
| Prostate | | | | | | | | 0/2 | 0/2 | | 0/2 |
| Brain | | | | | | | | 0/1 | 0/1 | | 0/1 |
| Parathyroid gland | | | | | | | | 0/1 | 0/1 | | 0/1 |
| Kidney | | | | | | | | 2/3 | 2/3 | | 2/3 |

a. The staining was regarded as positive if the percentage of cells stained $\geq$ 25% and staining intensity $\geq$ +.

b. Included fibroadenoma (2) and fibrocystic disease (1)

EP 0 369 816 A2

## TABLE 2

### THE REACTION OF BC1, BC2 and BC3 WITH PEPTIDES AND HMFG

| | No of Amino Acids | BC1 | | | BC2 | | | BC3 | | |
|---|---|---|---|---|---|---|---|---|---|---|
| | | Direct* Binding | Direct* Binding (BSA-pep) | Inhibition# Test | Direct* Binding | Direct Binding (BSA-pep) | Inhibition# Test | Direct* Binding | Direct* Binding (BSA-pep) | Inhibition# Test |
| P1-24 PDTRPAPGSTAPPAHGVTSAPDTR | (24) | - | 3+ | 4+ | 3+ | 3+ | 3+ | 4- | 3+ | 4+ |
| P21-24 PDTR | (4) | - | - | - | - | - | - | - | - | - |
| P20-24 APDTR | (5) | - | 2+ | 4+ | - | 2+ | 3+ | - | 3- | - |
| P19-24 SAPDTR | (6) | - | 2+ | 4+ | - | 2+ | 3+ | - | 3+ | 2+ |
| P18-24 TSAPDTR | (7) | - | 3+ | 4+ | - | 3+ | 3+ | - | 3+ | 3+ |
| P27-24 VTSAPDTR | (8) | - | 3+ | 4+ | - | 3+ | 3+ | - | 3+ | 4- |
| P16-24 GVTSAPDTR | (9) | - | 3+ | 4+ | - | 3+ | 3† | - | 3+ | 4- |
| P15-24 HGVTSAPDTR | (10) | - | 4+ | 4+ | 2+ | 4+ | 3+ | 3+ | 3+ | 4+ |
| P14-24 AHGVTSAPDTR | (11) | + | 4+ | 4+ | 3+ | 4+ | 3+ | 4+ | 3+ | 4+ |
| P14-23 AHGVTSAPDT | (10) | - | - | - | - | - | - | - | - | - |
| P1-15 PDTRPAPGSTAPPAH | (15) | - | - | 3+ | - | - | - | - | - | - |
| A+P1-15 APDTRPAPGSTAPPAH | (16) | 2+ | 3+ | 4+ | 2+ | 3+ | 3+ | 2+ | 3+ | 2+ |
| T4NI KTLVLGKEQSAELPCECY | (19) | - | - | - | - | - | - | - | - | - |
| HMFG | | 3+ | NT | 4+ | 4+ | NT | 3+ | 4+ | NT | 4+ |

\* - OD 0.0-0.5 + OD 0.5-1.0, 2+ OD 1.0-1.5
3+ OD 1.5-2.0, 4+ 2.0-2.2

# - 0.20% inhibition + 20-40% inhibition
2+ 40-60% inhibition 3+ 60-80% inhibition
4+ 80-100%

@ NT, not tested

Table 3. Reaction of synthetic peptides with mAbs 3C1, 3C2 and 3C3

| SYNTHETIC PEPTIDES AND HMFG | | | | | | 3C1 | | 3C2 | | 3C3 | |
|---|---|---|---|---|---|---|---|---|---|---|---|
| | 1 | 10 | 20 | 30 | 40 | DIR | INH. | DIR. | INH. | DIR. | INH. |
| | PDTRPAPGSTAPPAHGVTSAPDTRPAPGSTAPPAHGVTSA | | | | | | | | | | |
| | | | APDTR | | | NT[c] | 2+ | NT | 2+ | NT | + |
| | | | GPDTR | | | NT | - | NT | - | NT | - |
| | | | APKTR | | | NT | - | NT | - | NT | - |
| | | | FPKTR | | | NT | - | NT | - | NT | - |
| | | | ADVTR | | | NT | - | NT | - | NT | - |
| | | | AADTR | | | NT | - | NT | - | NT | - |
| | | | APESR | | | NT | - | NT | - | NT | - |
| P1-15 | PDTRPAPGSTAPPAH | | | | | - | 3+ | - | - | - | - |
| A-P1-15 | APDTRPAPGSTAPPAH | | | | | 2+ | 4+ | 2+ | 4+ | 2+ | 2+ |
| P1-24 | PDTRPAPGSTAPPAHGVTSAPDTR | | | | | + | 4+ | 3+ | 4+ | 3+ | 4+ |
| A-P1-24 | APDTRPAPGSTAPPAHGVTSAPDTR | | | | | + | 4+ | 3+ | 4+ | 2+ | 4+ |
| TSA-P1-24 | TSAPDTRPAPGSTAPPAHGVTSAPDTR | | | | | 3+ | 4+ | 3+ | 4+ | 3+ | 4+ |
| P13-32 | PAHGVTSAPDTRPAPGSTAP | | | | | 2+ | 4+ | 3+ | 4+ | 4+ | 4+ |
| C-P13-32 | CPAHGVTSAPDTRPAPGSTAP | | | | | 4+ | 4+ | 4+ | 4+ | 4+ | 4+ |
| T4N1 | KTLVLGKEQESAELPCECY | | | | | - | - | - | - | - | - |
| HMFGM | | | | | | 4+ | 4+ | 4+ | 4+ | 3+ | 4+ |

a. DIR., direct binding; INH., inhibition test,.

b. standards are used: for direct binding: Absorbance, 0≤-<0.5; 0.5≤+<1.0; 1.0≤2+<1.5; 1.5≤3+<2.0; 2.0≤4+<2.3, at antibody concentration of 75μg/al; for inhibition test:percent inhibition, 0≤-<20; 20≤+<40; 40≤2+<60; 60≤3+<80; 80≤4+<100, at competitor concentration, 0.04nM.

c. NT, not tested.

EP 0 369 816 A2

Table 4

| Analysis of epitope expression on BC3 -defined molecules using a double - determinant immunoassay. | | | | |
|---|---|---|---|---|
| Wells coated with antibody | Binding of $^{125}$I - antibody (cpm) | | | |
| | BC1 | BC2 | BC3 | 3E1.2 |
| BC3 (then treated with serum[a])<br>BC3 (then treated with buffer[b]) | $2 \times 10^3$<br>$4 \times 10^2$ | $11 \times 10^3$<br>$2 \times 10^2$ | $60 \times 10^3$<br>$9 \times 10^2$ | $14 \times 10^3$<br>$5 \times 10^2$ |

[a]. Serum of patient with disseminated breast cancer.
[b]. PBS/0.05% Tween 20.

Table 5

| Comparison of the serum tests in patients with breast cancer and in normal subjects. | | | | |
|---|---|---|---|---|
| | % Subjects with antigen level above cut off[a] | | | |
| | Normal | | Stage III/IV [b] | |
| MSA (3E1.2) | 3/66 | (5%) | 37/39 | (94%) |
| BC3/BC2 | 4/66 | (6%) | 34/38 | (87%) |
| BC3/3E1.2 | 2/25 | (8%) | 17/21 | (81%) |
| MSA and BC3/BC2 | 3/66 | (5%) | 39/39 | (100%) |
| MSA and BC3/3E1.2 | 2/25 | (8%) | 21/21 | (100%) |

[a]. Cut off levels of 300 I.U., 100 units, 80 units were used respectively for MSA, BC3/BC2 and BC3/3E1.2. Arbitary units were used in the quantification of the antigen level in each respective assay.
[b]. Criteria of staging is in accord with that reported by Beahrs et al (18).

**Claims**

1. A monoclonal antibody which specifically binds to the amino acid sequence Ala Pro Asp Thr Arg (APDTR) of the repeat sequence of the human polymorphic epithelial mucin (HPEM).

2. A monoclonal antibody according to claim 1, which further binds to mucin produced by both tumourigenic and non-tumourigenic epithelial cells.

3. A monoclonal antibody which specifically binds to the amino acid sequence:

X A′ P D′ T′ R X

wherein:

A′ is A, P or S;

D′ is D or N;

T′ is any amino acid; and

X represents 1 to $1\times10^4$ amino acids which may be the same, or may be a combination of any known L-amino acids found in protein molecules.

4. A monoclonal antibody which specifically binds to the epitope APDTR or A′PD′T′R, wherein A′, D′ and T′ are as defined in claim 3, in a peptide or protein.

5. A monoclonal antibody according to claim 3, wherein said amino acid sequence is selected from:

p 21 - 24

p 20 - 24

p 19 - 24

p 17 - 24

p 16 - 24

p 15 - 24

p 14 - 24

p 14 - 23

p 13 - 32

p 1 - 15

TSA p 1 - 24

p 13 - 32

Cp 13 - 32

Ap 1 - 15

wherein numbers following the letter "p" refer to amino acids 1-40 of the two amino acid repeat sequence of HPEM.

6. An antibody according to any preceding claim which is of rodent, rabbit, horse, sheep, donkey or human origin.

7. An antibody according to any preceding claim wherein the rodent is mouse.

8. An antibody according to any preceding claim which is a rodent/human antibody having an antigen binding region of rodent origin and a constant region of human origin.

9. An antigen binding fragment of an antibody according to any preceding claim.

10. An antibody according to any preceding claim which is labelled with a reporter group.

11. An antibody according to claim 10, wherein said reporter group is selected from a radioisotope, enzyme, metal, or fluorophore.

12. An antibody according to any preceding claim which is linked, conjugated or associated with a cytotoxic or tumourigenic agent.

13. A hybridoma cell line which secretes an antibody according to any one of claims 1 to 9.

14. A hybridoma cell line according to claim 13 which is of rodent, rabbit, horse, sheep, donkey or human origin.

15. A hybridoma cell line according to claim 13 which is a hybrid between cells of different species.

16. A method for detecting HPEM which comprises contacting a biological sample with a monoclonal antibody according to any one of claims 1 to 12 and thereafter detecting and/or quantitating antibody binding.

17. A method for detecting HPEM in a biological sample which comprises the steps of:

(i) contacting a biological sample with a monoclonal antibody according to any one of claims 1 to 11;

(ii) adding the mix of step (i) to a support having immobilized thereon a peptide sequence reactive with the monoclonal antibody of step (i); and

19

(iii) thereafter detecting and or quantitating antibody binding.

18. A method according to claim 17, wherein antibody binding is detected with an anti-antibody which binds to the monoclonal antibody bound to the peptide sequence, and which carries a reporter group.

19. A method according to claim 16 or 17, wherein said biological sample is selected from serum, plasma, milk, urine, faeces, ascites, tissue extracts, cells and cell supernatants.

20. A method for detecting tumours in-vivo which comprises injecting a subject with a monoclonal antibody as claimed in claim 10, and thereafter detecting antibody binding to tissue using detection means.

21. A synthetic, non-naturally occuring peptide or protein which contains the epitope APDTR or $S'A'PD'T'R$, or multiple copies thereof, wherein; $S'$ is A, D, E, F, G, K, S or P; $A'$ is A, P or S; $D'$ is D or N; and $T'$ is any amino acid found in peptides or proteins.

22. A synthetic, non-naturally occuring peptide or protein selected from:

p 21 - 24
p 20 - 24
p 19 - 24
p 18 - 24
p 17 - 24
p 16 - 24
p 15 - 24
p 14 - 24
p 1 - 15
TSA p 1 - 20
Ap 1 - 20
p 13 - 32
p 13 - 32
Cp 13 - 32

wherein p refers to amino acids of two twenty amino acid repeats of HPEM.

23. A synthetic, non-naturally occuring peptide or protein according to claim 21, having a sequence selected from:

$[S'A'PD'T'R]^n$
$[APDTR]^n$
$[SAPDTR]^n$

wherein X represents any amino acid, and n represents 1 to $1 \times 10^6$ and $S'$, $A'$, $D'$ and $T'$ are as defined in claim 21.

24. A vaccine which comprises a peptide according to any one of claims 21 to 23, in association with a pharmaceutically acceptable carrier or excipient, and/or adjuvant.

25. A vaccine for the treatment of epithelial adenocarcinoma which comprises a peptide or protein capable of eliciting antibodies and T-cells which specifically bind to the epitope APDTR of human polymorphic epithelial mucin (HPEM), in combination with a pharmaceutically acceptable carrier or excipient, and/or adjuvant.

26. A vaccine according to claim 25, wherein said peptide or protein is selected from:

$[S'A'PD'T'R]^n$-Y
$[SAPDTR]^n$-Y
$[APDTR]_1{}^n$-y; or
$[TS'APDT'RPAPGSTAPPAHGVTS'APDT'R]^n$;

wherein; n is 1 to $1 \times 10^6$, and y is an adjuvanting sequence or a carrier molecule, and $S'$, $A'$, $D'$ and $T'$ are as defined in claim 21.

27. A vaccine according to claim 26, wherein said adjuvanting sequence is selected from PPD, BCG or MPD or analogues thereof.

28. A vaccine according to claim 25 or 26, wherein said carrier molecule is a protein.

29. A vaccine according to claim 28, wherein said carrier is diptheria or tetanus toxoid.

30. A therapeutic composition for the treatment of adenocarcinoma which comprises a monoclonal antibody according to any one of claims 1 to 11 either alone or in association with a pharmaceutically acceptable carrier or excipient.

31. A therapeutic compoistion according to claim 30 which is a human antibody, or a humanized antibody containing hypervariable regions from another species.

32. A therapeutic composition according to claim 30 or 31 wherein said monoclonal antibody is conjugated or associated with a cytotoxic agent.

33. A therapeutic composition according to claim 32 wherein said cytotoxic agent is selected from the cytotoxic sub-unit of ricin, or tetanus toxoid or other cytotoxic peptides or proteins; or cytotoxic drugs, selected from melphalan, n-acetyl melphalan, andriamycin, bleomycin, cisplatin, daunorubicin, actinomycin, cyclophosphamide, 5-fluorouracil, methotrexate; and salts or derivatives thereof.

EP 0 369 816 A2

**FIGURE 1**

| | | | | | | | | | | | | | | | | | | | |
|---|---|---|---|---|---|---|---|---|---|---|---|---|---|---|---|---|---|---|---|
| 1 | | | | 5 | | | | | 10 | | | | | 15 | | | | | 20 |
| CCG | GAC | ACC | AGG | CCG | GCC | CCG | GGC | TCC | ACC | GCC | CCC | CCA | GCC | CAC | GGT | GTC | ACC | TCG | GCC |
| Pro | Asp | Thr | Arg | Pro | Ala | Pro | Gly | Ser | Thr | Ala | Pro | Pro | Ala | His | Gly | Val | Thr | Ser | Ala |
| P | D | T | R | P | A | P | G | S | T | A | A | P | A | H | G | V | T | S | A |

## FIGURE   2

| Amino acid symbols | | |
|---|---|---|
| Amino acid | Three-letter symbol | One-letter symbol |
| Alanine | Ala | A |
| Arginine | Arg | R |
| Asparagine | Asn | N |
| Aspartic acid | Asp | D |
| Asn and/or Asp | Asx | B |
| Cysteine | Cys | C |
| Glutamine | Gln | Q |
| Glutamic acid | Glu | E |
| Gln and/or Glu | Glx | Z |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

FIGURE 3A

FIGURE 3B